# EUROPEAN PATENT SPECIFICATION

(11) **EP 4 332 289 B1**
(45) Date of publication and mention of the grant of the patent: **05.03.2025**
(21) Application number: 21945221.6
(22) Date of filing: 11.06.2021
(51) Int. Cl.: A61F 13/511, D04H 3/007, D04H 3/02, D04H 3/147, D04H 3/16

(54) **NONWOVEN FABRIC FOR ABSORBENT ARTICLES AND METHOD FOR PRODUCING SAME**
VLIESSTOFF FÜR SAUGFÄHIGE ARTIKEL UND VERFAHREN ZUR HERSTELLUNG DAVON
NON-TISSÉ POUR ARTICLES ABSORBANTS ET SON PROCÉDÉ DE PRODUCTION

(43) Date of publication of application: 06.03.2024
(73) Proprietor: Unicharm Corporation, Shikokuchuo-shi, Ehime 799-0111 (JP)
(72) Inventor: MITSUNO, Satoshi, Kanonji-shi, Kagawa 769-1602 (JP)
(74) Representative: Dolleymores
(86) International application number: PCT/JP2021/022399
(87) International publication number: WO 2022/259551

(56) References cited:
- JP-A- 2003 268 667
- JP-A- 2008 025 082
- JP-A- 2008 025 084
- JP-A- 2013 224 500
- JP-A- 2013 231 249
- JP-A- 2015 113 548
- JP-A- H08 509 786
- US-A1- 2003 203 162
- No further relevant documents disclosed

## Description

### FIELD

The present invention relates to a nonwoven fabric for an absorbent article, and a method of manufacturing the same.

### BACKGROUND

A nonwoven fabric having a plurality of protruding and recessed portions on its surface is known. The nonwoven fabric is used, for example, for a top sheet of a disposable diaper.

Patent Literature 1 discloses a nonwoven fabric having a longitudinal direction and a lateral direction and formed by blowing a fluid mainly consisting of a gas to a fiber assembly, the nonwoven fabric including a plurality of low basis weight portions formed along the longitudinal direction, and a plurality of high basis weight portions formed so as to be adjacent to and along each of the plurality of low basis weight portions, in which a basis weight of each of the plurality of low basis weight portions is lower than a basis weight of each of the plurality of high basis weight portions.

According to Patent Literature 1, it is disclosed that at least the basis weight of a fiber web can be adjusted by blowing a gas from the upper surface side to the fiber web that is supported from the lower surface side by a predetermined air permeable support member so as to move fibers forming the fiber web, and thus a nonwoven fabric that easily allows permeation of a liquid can be provided.

### [CITATION LIST]

### [PATENT LITERATURE]

Patent Literature 1: Japanese Unexamined Patent Publication No. 2008-025079
Patent Literature 2: JP H08 509786A

### SUMMARY

### [TECHNICAL PROBLEM]

For example, in a case where the nonwoven fabric described in Patent Literature 1 is applied to a top sheet of a disposable diaper, excreted bodily fluid easily permeates the low basis weight portion, and the bodily fluid that has permeated the low basis weight portion and is absorbed by the absorbent body can be visually recognized through the low basis weight portion. However, since the nonwoven fabric is formed of short fibers, the nonwoven fabric is easily deformed in the thickness direction. The nonwoven fabric that is easily deformed in the thickness direction easily becomes thin when a load is applied in the thickness direction while the diaper is put on. In a case where the nonwoven fabric that easily becomes thin as described above is applied to the top sheet of a disposable diaper, the thickness of the plurality of high basis weight portions is reduced and expanded in the planar direction by receiving a load in the thickness direction. When the plurality of high basis weight portions are expanded in the planar direction, the range of the low basis weight portions having a lower basis weight becomes narrow by that amount, and as a result, the visibility is easily lowered.

Patent Literature 2 discloses a spunbond nonwoven fabric.

An aspect of the present invention is to provide a nonwoven fabric having excellent visibility in the thickness direction.

### [SOLUTION TO PROBLEM]

The present invention is
a nonwoven fabric for an absorbent article, having a first direction, a second direction, and a thickness direction that are orthogonal to each other and formed of continuous fibers, the nonwoven fabric comprising:
a plurality of ridge portions that extend along the first direction; and
a plurality of groove portions that extend along the first direction and have a basis weight lower than that of the plurality of ridge portions, wherein
each of the plurality of ridge portions and each of the plurality of groove portions are alternately located in the second direction, and
each of the plurality of ridge portions and each of the plurality of groove portions have a plurality of joining portions in which intersections of the continuous fibers are thermally joined, characterized in that
each of the plurality of ridge portions has a plurality of recessed portions recessed in the thickness direction, and a base formed of the continuous fibers at a bottom of each of the plurality of recessed portions.

### [ADVANTAGEOUS EFFECT OF THE INVENTION]

The nonwoven fabric according to the present invention has excellent visibility in the thickness direction.

### BRIEF DESCRIPTION OF DRAWINGS

FIG. 1 is a perspective view schematically showing a nonwoven fabric according to an embodiment.
FIG. 2 is a cross-sectional view orthogonal to a first direction of the nonwoven fabric according to the embodiment.
FIG. 3 is a perspective view schematically showing a wire mesh used in a method of manufacturing the nonwoven fabric according to the embodiment.
FIG. 4 is a cross-sectional view of the wire mesh orthogonal to an MD direction.
FIG. 5 is a plan view schematically showing the absorbent article according to the embodiment.
FIG. 6A is a micrograph (20 times) of the nonwoven fabric.
FIG. 6B is a micrograph (100 times) of the nonwoven fabric in which a part of FIG. 6A is enlarged.
FIG. 6C is a micrograph (300 times) of the nonwoven fabric in which a part of FIG. 6B is enlarged.
FIG. 6D is a micrograph (50 times) of a cross section orthogonal to the first direction of the nonwoven fabric.

### DESCRIPTION OF EMBODIMENTS

A first aspect of the present invention relates to a nonwoven fabric for an absorbent article, having a first direction, a second direction, and a thickness direction that are orthogonal to each other and formed of continuous fibers, the nonwoven fabric comprising:
a plurality of ridge portions that extend along the first direction; and
a plurality of groove portions that extend along the first direction and have a basis weight lower than that of the plurality of ridge portions, wherein
each of the plurality of ridge portions and each of the plurality of groove portions are alternately located in the second direction, and
each of the plurality of ridge portions and each of the plurality of groove portions have a plurality of joining portions in which intersections of the continuous fibers are thermally joined. Since the nonwoven fabric is formed of continuous fibers, and each of the plurality of ridge portions and each of the plurality of groove portions has a plurality of joining portions in which the intersections of the continuous fibers are thermally joined, the shape is easily retained, and a change in thickness in a state where a load is applied in the thickness direction is small compared with a nonwoven fabric formed of short fibers. Since the nonwoven fabric has a plurality of joining portions in which intersections of the continuous fibers are thermally joined, the shape is easily retained. Therefore, in a case where a load is applied in the thickness direction, the thickness of the ridge portions is less likely to be changed. In such a case, since the length of the ridge portion in the second direction is less likely to be changed, the length of the groove portion in the second direction is less likely to be changed. That is, since the structures of the ridge portions and the groove portions are less likely to be changed and the length of the groove portion in the second direction is less likely to be changed in the nonwoven fabric, the range in which visibility can be obtained is less likely to be changed. Therefore, the groove portions have excellent visibility in the thickness direction through the groove portions. For example, in a case where the nonwoven fabric is applied to the outermost layer of a disposable diaper, even in a case where the nonwoven fabric receives a load over time after the diaper is put on, the nonwoven fabric has excellent visibility in the thickness direction through the groove portions. Since the ridge portions and the groove portions are alternately located in the second direction, the nonwoven fabric has excellent visibility through the groove portions in a wide range in the first direction and the second direction. Incidentally, in the nonwoven fabric formed of short fibers, in a case where a load is applied in the thickness direction, the ridge portion easily becomes thin. When the ridge portion becomes thin by receiving the load in the thickness direction, the length of the ridge portion in the second direction is extended, and the length of the groove portion in the second direction becomes narrow by that amount. When the width of the groove portion becomes narrow, a range in which visibility can be obtained becomes narrow. Therefore, the visibility of the groove portions of the nonwoven fabric formed of short fibers and having ridge portions and groove portions is easily deteriorated.

The nonwoven fabric can be for a liquid-permeable sheet of the absorbent article. The absorbent article is exemplified by an absorbent article including a liquid-permeable sheet, an absorbent body, and a liquid-impermeable sheet in this order. The nonwoven fabric is applied to, for example, a liquid-permeable sheet of a disposable diaper, and since there is little change in thickness while the diaper is put on, a state where a space between the absorbent body and the skin of a user is retained is easily maintained. Due to that, the nonwoven fabric is excellent in suppressing a wet feeling. Since the ridge portions and the groove portions are alternately located in the second direction, the nonwoven fabric is excellent in suppressing a wet feeling by retaining a space between the absorbent body and the skin of the user in a wide range in the first direction and the second direction. Incidentally, since the shape of the nonwoven fabric formed of short fibers is easily changed and a change in thickness in a state where a load is applied in the thickness direction is large, the nonwoven fabric easily becomes thin. Therefore, since the nonwoven fabric formed of short fibers is difficult to maintain a state where a space between the absorbent body and the skin of the user is retained, a wet feeling easily occurs.

The nonwoven fabric can be for an exterior sheet of the absorbent article. The absorbent article is exemplified by an absorbent article including a liquid-permeable sheet, an absorbent body, a liquid-impermeable sheet, and an exterior sheet in this order. The absorbent article may be arranged on an indicator (preferably between the absorbent body and the liquid-impermeable sheet) and on a pattern (preferably the inner surface or outer surface of the liquid-impermeable sheet). The nonwoven fabric can be applied to, for example, an exterior sheet of a disposable diaper. In the nonwoven fabric, even in a case where a load is applied in the thickness direction, the plurality of ridge portions are less likely to collapse, and the length of the plurality of groove portions in the second direction is maintained in a state where the basis weight is small. Therefore, excellent breathability and visibility in the plurality of groove portions are maintained. Therefore, in an absorbent article in which the nonwoven fabric is applied to the exterior sheet and the surface on which the plurality of ridge portions and the plurality of groove portions are located is arranged on the non-skin side, excellent breathability and visibility can be obtained after the absorbent article is put on. In addition, in the absorbent article in which the nonwoven fabric is applied to the exterior sheet and the surface on which the plurality of ridge portions and the plurality of groove portions are located is arranged on the skin side, a space is formed between the nonwoven fabric and the liquid-impermeable sheet, which makes it difficult to transmit moisture to clothing, sheets, or the like.

In the nonwoven fabric according to the present invention,
each of the plurality of ridge portions has a plurality of recessed portions recessed in the thickness direction, and a base formed of the continuous fibers at a bottom of each of the plurality of recessed portions.

In the nonwoven fabric, since the nonwoven fabric has the plurality of groove portions and the plurality of recessed portions that are recessed in the thickness direction in the ridge portions, the area where the skin of the user touches the nonwoven fabric is small compared with a nonwoven fabric having no recessed portions. In the nonwoven fabric, for example, in a case where the nonwoven fabric is applied to a liquid-permeable sheet of a disposable diaper, the base absorbs excreted bodily fluid and more reliably prevents the bodily fluid absorbed by the absorbent body from returning into the recessed portions. Therefore, the nonwoven fabric is excellent in suppressing a wet feeling. In addition, in the nonwoven fabric, for example, in a case where the nonwoven fabric is applied to an exterior sheet of a disposable diaper, the surface on which the plurality of ridge portions and the plurality of groove portions are located may be arranged on the non-skin side. In this case, since the contact area of the surface of the nonwoven fabric is further reduced by the amount corresponding to the presence of the recessed portions, the nonwoven fabric can give a soft tactile feel and has a fabric-like complex surface shape. In addition, in the nonwoven fabric, in a case where the surface on which the plurality of ridge portions and the plurality of groove portions are located is arranged on the skin side, a space formed between the nonwoven fabric and the liquid-impermeable sheet is further increased by the amount corresponding to the recessed portions, which makes it difficult to transmit moisture to clothing, sheets, or the like.

In an embodiment,
each of the plurality of ridge portions has a bundle-shaped portion joined at the plurality of joining portions in a state where the continuous fibers are oriented along the first direction on one side of at least a part of the plurality of recessed portions in the second direction. The bundle-shaped portion has a small inter-fiber distance between the continuous fibers and is less likely to be deformed when receiving a load. Therefore, the nonwoven fabric having the bundle-shaped portion easily retains the shape of the recessed portion in which there is little change in thickness when the absorbent article is put on.

In an embodiment,
a thickness of each of the plurality of groove portions is smaller than a thickness of the base. In the nonwoven fabric, since the thickness of each of the plurality of groove portions is smaller than the thickness of the base, for example, in a case where the nonwoven fabric is applied to a liquid-permeable sheet or an exterior sheet of a disposable diaper, the groove portion has excellent visibility in the thickness direction. Therefore, the difference from the ridge portion where almost no visibility in the thickness direction is obtained is remarkable. Therefore, the nonwoven fabric has excellent visibility.

In an embodiment,
the continuous fibers are conjugated fibers containing an olefin-based resin, which means that joining portions can be formed even at a low heat treatment temperature, and thus joining portions can be more efficiently formed in the entire nonwoven fabric. Since the joining portions are formed in the entire nonwoven fabric, even in a case where a load is applied in the thickness direction after the absorbent article is put on, the shape is easily retained, which makes it difficult to change the structures of the ridge portions and the groove portions.

In an embodiment,
an average fiber diameter of the continuous fibers is 15 µm or more and 30 µm or less, and an average basis weight of the nonwoven fabric is 10 g/m² or more and 40 g/m² or less. Therefore, when a load is applied in the thickness direction after the absorbent article is put on, the structures of ridge portions and groove portions that easily retain the shape can be more reliably obtained.

In an embodiment,
the first direction is arranged in a lengthwise direction of the absorbent article. In the nonwoven fabric, when the groove portions and the ridge portions are arranged along the lengthwise direction of the absorbent article, the nonwoven fabric has excellent visibility in the lengthwise direction. In addition, in a case where the nonwoven fabric is applied to a liquid-permeable sheet of a disposable diaper, the bodily fluid is easily diffused in the lengthwise direction of the absorbent article. Therefore, since the nonwoven fabric prevents the bodily fluid from staying at one place by diffusing the bodily fluid in the lengthwise direction, the nonwoven fabric is excellent in suppressing a wet feeling.

In an embodiment,
the first direction is arranged in a width direction of the absorbent article. In the nonwoven fabric, when the groove portions and the ridge portions are arranged along the width direction of the absorbent article, the nonwoven fabric has excellent visibility in the width direction. In addition, in a case where the nonwoven fabric is applied to a liquid-permeable sheet of a disposable diaper, the bodily fluid is easily diffused in the width direction of the absorbent article. Therefore, since the nonwoven fabric prevents the bodily fluid from staying at one place by diffusing the bodily fluid in the width direction, the nonwoven fabric is excellent in suppressing a wet feeling.

In one embodiment,
each of the plurality of ridge portions has a plurality of protrusion portions that protrude toward each of the plurality of adjacent groove portions and are arranged side by side with spaces along the first direction. In this case the ridge portions are less likely to collapse in a case where a load is applied in the thickness direction. Therefore, in a case where the nonwoven fabric is applied to, for example, a liquid-permeable sheet or an exterior sheet of a disposable diaper, a state where the shape of the ridge portions is retained is easily maintained, the ridge portions do not block the groove portions, and thus visibility is excellent.

In one embodiment, the
fiber orientation of each of the plurality of ridge portions along the first direction is higher than fiber orientation of each of the plurality of groove portions along the first direction, which means that a distance between the intersections of the continuous fibers is short, and a distance between the plurality of joining portions is short. Therefore, the nonwoven fabric easily retains the shape of the plurality of ridge portions.

In one embodiment,
the nonwoven fabric is a spunbond nonwoven fabric. When the nonwoven fabric is a spunbond nonwoven fabric, in a case where the continuous fibers are moved by an airflow during manufacture, the continuous fibers are moved across a certain length due to the continuous fibers having a long fiber length, and further moved along with adjacent continuous fibers. Therefore, the continuous fibers are easily affected by the airflow in the deposition step. Accordingly, when each of the continuous fibers is moved, a portion having a small inter-fiber distance is easily formed. In the continuous fibers, the distance between the intersections of the continuous fibers is short, and the distance between the plurality of joining portions is short. Therefore, the nonwoven fabric easily retains the shape of the plurality of ridge portions. Incidentally, in a case where the short fibers of a nonwoven fabric formed of short fibers, for example, an air-laid nonwoven fabric are moved by an airflow during manufacture, since the fiber length is short, each short fiber is independently moved and has little influence on other fibers. Therefore, the short fibers are less likely to be affected by the airflow in the deposition step. Accordingly, since the short fibers have a small amount of moving fibers, the randomly deposited state is easily maintained, and thus the inter-fiber distance is larger than that in a case of continuous fibers. Since the nonwoven fabric formed of short fibers has a large inter-fiber distance, the nonwoven fabric is easily deformed when receiving a load, and has a large change in thickness while the absorbent article is put on. Therefore, since the nonwoven fabric formed of short fibers is difficult to maintain a state where a space between the absorbent body and the skin of the user is retained, a wet feeling is easily caused. In addition, the nonwoven fabric formed of short fibers has a large amount of change in the thickness direction before and after putting on the absorbent article. Accordingly, when the nonwoven fabric receives a load in the thickness direction or when the thickness is recovered, the structures of the ridge portions and the groove portions are easily changed. Therefore, in the nonwoven fabric formed of short fibers, since the upper side and the lower side of the nonwoven fabric in the thickness direction are easily displaced in the planar direction, the basis weight of the groove portion is easily changed, and thus the visibility is easily deteriorated.

A second aspect of the invention relates to a method of manufacturing the nonwoven fabric as defined in claims 1 to 12, the method comprising:
a step of, while sucking the continuous fibers from a lower side of a wire mesh having a transport direction, a transverse direction, and a height direction that are orthogonal to each other, depositing the continuous fibers on the wire mesh; and
a step of thermally joining the continuous fibers deposited on the wire mesh, wherein
the wire mesh includes
   a mesh main body, and
   a plurality of ribs that extend along the transport direction, are arranged on the mesh main body with predetermined spaces in the transverse direction, and protrude toward the height direction. In the method of manufacturing the nonwoven fabric, the continuous fibers are deposited on the wire mesh by being sucked from the lower side of the wire mesh. Some of the continuous fibers preferentially are collected between the ribs in which the ribs of the mesh main body are not formed. When some of the continuous fibers are preferentially collected between the ribs, the continuous fibers become dense and ridge portions having a high basis weight are formed. Further, on the ribs, the continuous fibers become sparse and groove portions having a low basis weight are formed. Further, by thermally joining the continuous fibers in a state of being deposited on the wire mesh, the shapes of the ridge portions and the groove portions are maintained. Therefore, in the method of manufacturing the nonwoven fabric, a nonwoven fabric formed of continuous fibers, including a plurality of ridge portions and a plurality of groove portions, and having a plurality of joining portions in which intersections of the continuous fibers are thermally joined can be manufactured.

In an embodiment,
a height of the rib is 1.0 mm or more. When the height of the rib is 1.0 mm or more, the continuous fibers are less likely to remain on the rib, and the continuous fibers are accumulated between the ribs. Therefore, a ridge portion having a predetermined thickness can be formed. Therefore, in the method of manufacturing the nonwoven fabric, a nonwoven fabric having a plurality of ridge portions and a plurality of groove portions can be more reliably manufactured.

In an embodiment,
a total length of lengths of each of the plurality of ribs in the transverse direction is 50% or less of a length of the mesh main body in the transverse direction, meaning that a sufficient amount of airflow of gas passes through the mesh main body excluding the ribs. Therefore, in the method of manufacturing the nonwoven fabric, since the continuous fibers can be stably deposited on the wire mesh, a nonwoven fabric having a plurality of ridge portions and a plurality of groove portions can be more reliably manufactured.

In an embodiment,
each of the plurality of ribs has a plurality of bulging portions that bulge in the transverse direction. This means that in the bulging portions, the continuous fibers become sparse, and portions between the bulging portions are protrusion portions protruding from the ridge portions toward the groove portions. Therefore, in the method of manufacturing the nonwoven fabric, a nonwoven fabric having protrusion portions in the ridge portions can be manufactured.

In an embodiment,
an outer diameter of a wire that forms the wire mesh is smaller than a height of the rib. This means that the rib is higher than the wire even in the portion in which the wires of the wire mesh are overlapped, and thus groove portions are formed. Therefore, in the method of manufacturing a nonwoven fabric, a nonwoven fabric having ridge portions and groove portions can be more reliably manufactured. Further, since the portion in which the wires are overlapped is smaller than the rib, the continuous fibers are easily deposited on the network of the wire mesh, and thus a ridge portion having a recessed portion can be formed.

In an embodiment,
the continuous fibers are deposited on the wire mesh at a fiber speed of 1000 m/min or more and 4500 m/min or less. When the fiber speed of the continuous fiber is 1000 m/min or more, the continuous fibers are easily deposited on the wire mesh, and the ridge portions are easily formed. Further, when the fiber speed of the continuous fiber is 4500 m/min or less, the orientation of the continuous fibers can be prevented from being disturbed on the wire mesh, and the ridge portions are easily formed. Therefore, when the number of continuous fibers is within the above range, a nonwoven fabric having a plurality of ridge portions and a plurality of groove portions can be efficiently manufactured.

Hereinafter, a nonwoven fabric for an absorbent article according to an embodiment will be described. In the present specification, the term "along a predetermined direction" with respect to an object means that the object extends so as to have an intersection angle of less than ± 90° with the predetermined direction (for example, the first direction, the second direction, and the like). In addition, in a case where the object extends in a direction along the predetermined direction, the object has an intersection angle with the predetermined direction, preferably 60° or less, more preferably 45° or less, still more preferably 30° or less, and even more preferably 20° or less, and still even more preferably 5° or less. As appropriate, the orthogonal coordinates shown in the drawings are defined as follows: x represents a first direction; y represents a second direction, z represents a thickness direction, MD represents a transport direction; CD represents a transverse direction; H represents a height direction; further, L represents a lengthwise direction; and W represents a width direction.

### (Configuration of Nonwoven Fabric)

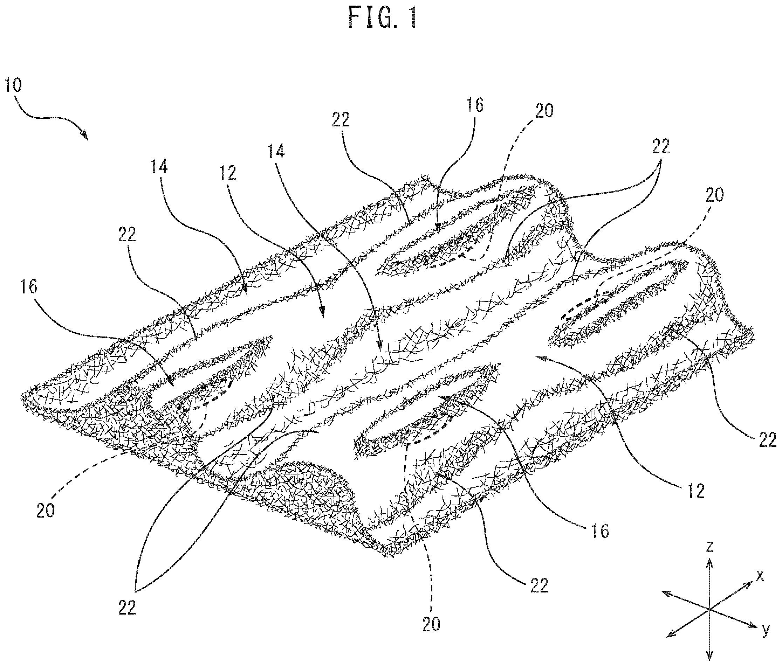

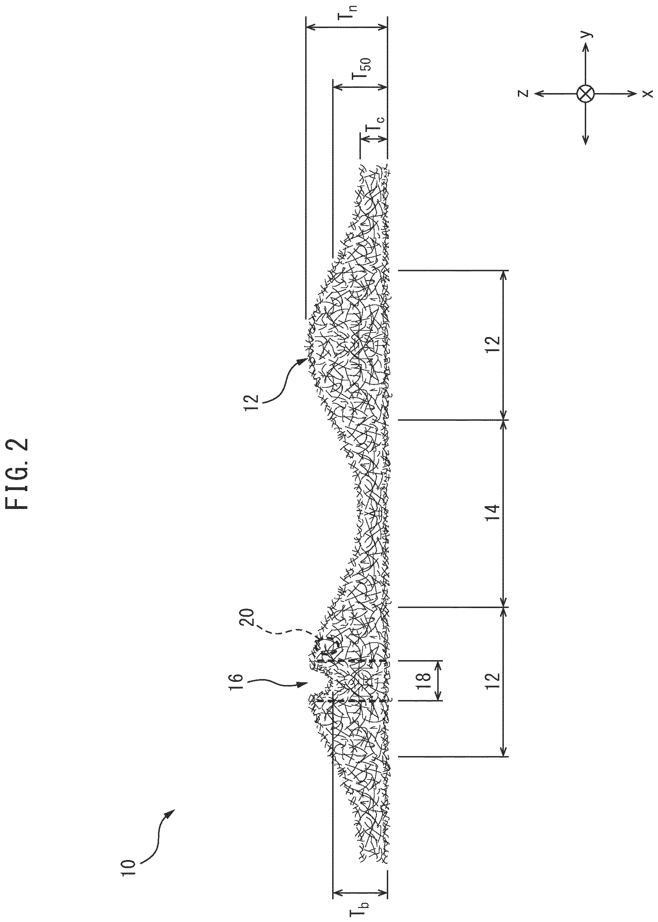

A nonwoven fabric 10 for an absorbent article shown in FIG. 1 has a first direction x, a second direction y, and a thickness direction z that are orthogonal to each other and is formed of continuous fibers. The nonwoven fabric 10 is not particularly limited as long as the nonwoven fabric is formed of continuous fibers, and for example, there is provided a spunbond nonwoven fabric. The continuous fibers are not particularly limited as long as the fibers can be melted by heating and thermally joined. As such heat-fusible fibers, there are provided polyolefin-based single fibers such as polyethylene, polypropylene, and polyvinyl alcohol fibers, and core-sheath type conjugated fibers containing an olefin-based resin such as polyethylene terephthalate (core portion)/polyethylene (sheath portion), polyethylene terephthalate (core portion)/polypropylene (sheath portion), and polypropylene (core portion)/polyethylene (sheath portion) fibers. The conjugated fiber may be a hydrophobic fiber or a fiber obtained by subjecting the fiber to a hydrophilization treatment with a hydrophilic oil agent or the like. The hydrophilization treatment can be performed, for example, by kneading a hydrophilizing agent into a resin constituting the fiber, applying the hydrophilizing agent to the surface of the fiber, and the like. Further, the conjugated fiber may be a side-by-side-type fiber containing an olefin-based resin as described above. These fibers may be used alone or two or more types of fibers may be used in combination. It is preferable that the continuous fiber contain a thermoplastic resin fiber having a lower melting point. The average fiber diameter of the continuous fibers may be 15 µm or more and 30 µm or less. Since the nonwoven fabric 10 is formed of continuous fibers, it is preferable that the surface of the nonwoven fabric 10 should not include the end portions of the continuous fibers. The thin lines in FIGS. 1 and 2 schematically represent fibers in the nonwoven fabric 10, and the direction, length, and diameter of the fibers do not mean the direction, length, and diameter of the continuous fibers in the nonwoven fabric 10.

The average fiber diameter of the continuous fibers can be measured as follows. First, a 10 mm × 10 mm sample of the nonwoven fabric is cut out and arranged on a glass slide. Next, an appropriate amount of glycerin is added dropwise to the sample to make the entire sample in a state of being immersed in the glycerin, and a cover glass is placed thereon. Next, the sample is observed at a magnification of 1000 times using a known optical microscope (for example, a VHC-100 Digital Microscope Lens VH-Z450, manufactured by KEYENCE CORPORATION), the fiber diameter of the fibers exposed on the surface of the sample is measured at 50 places, and an average value is set as an average fiber diameter.

As the outer shape, various dimensions, average basis weight, and the like of the nonwoven fabric 10, any outer shape, various dimensions, average basis weight, and the like according to various configurating members of the absorbent article to which the nonwoven fabric 10 is applied can be employed. For example, the average basis weight of the nonwoven fabric 10 may be from 10 g/m² or more and 40 g/m² or less.

The average basis weight is measured by the following method. First, five samples having a predetermined size (for example, 100 mm × 100 mm) are cut out from the nonwoven fabric. Next, the mass of each of the five-cut samples is measured with a direct-reading balance (for example, electronic balance HF-300, manufactured by Kensei Kogyo Co., Ltd.). Then, the mass per unit area (g/m²) of the nonwoven fabric is calculated from the average value of the masses of the five samples and the calculated value is used as the average basis weight of the nonwoven fabric.

The average thickness of the nonwoven fabric 10 is, for example, 0.1 mm to 3 mm. The average thickness of the nonwoven fabric 10 refers to the average value of five measured values obtained by, in a preparation state (temperature: 23±2°C, relative humidity: 50±5%), pressing five different portions of the nonwoven fabric by using FS-60DS [measurement surface: 50.5 mm (diameter), measurement pressure: 3 gf/cm² (0.3 kPa)] manufactured by Daiei Kagaku Seiki Mfg. Co., Ltd., and measuring the thickness at each portion after 10 seconds from the application of pressure.

The breaking strength per average basis weight of the nonwoven fabric 10 is not particularly limited as long as the effect of the present invention is not inhibited, and any breaking strength corresponding to the desired flexibility, strength, and the like can be employed. The breaking strength per average basis weight of the nonwoven fabric 10 in the first direction x can be, for example, 0.3 [(N/50 mm)/(g/m²)] or more and 3.0 [(N/50 mm)/(g/m²)] or less. The breaking strength per average basis weight of the nonwoven fabric 10 in the second direction y can be, for example, 0.2 [(N/50 mm)/(g/m²)] or more and 1.2 [(N/50 mm)/(g/m²)] or less.

The average breaking strength per average basis weight is defined as a value obtained by dividing the measured average breaking strength by the average basis weight. The average breaking strength is measured using an autograph, AG-1, manufactured by Shimadzu Corporation. First, five samples each having a size of a length of 150 mm in the first direction x and a length of 50 mm in the second direction y, and five samples each having a size of a length of 50 mm in the first direction x and a length of 150 mm in the second direction y are prepared. Then, the breaking strengths of the composite sheet in the first direction x and the second direction y are respectively measured at a chuck-to-chuck distance of 100 mm and a tensile rate of 100 mm/min. Then, the average value of the breaking strengths of each of the five samples is defined as the average breaking strength of each sample. The term "N/50 mm" means the breaking strength (N) per 50 mm width. It should be noted that the breaking strength means the strength at the maximum point load.

The nonwoven fabric 10 includes a plurality of ridge portions 12 and a plurality of groove portions 14. The plurality of ridge portions 12 and the plurality of groove portions 14 are located on one surface of the nonwoven fabric 10 in the thickness direction z, in a case of FIG. 1, an upper surface. The plurality of ridge portions 12 protrude from one surface of the nonwoven fabric 10 in the thickness direction z and extend along the first direction x. The plurality of groove portions 14 extend along the first direction x and have a basis weight lower than that of the plurality of ridge portions 12. Each of the plurality of ridge portions 12 and each of the plurality of groove portions 14 are alternately located in the second direction y. The plurality of ridge portions 12 are arranged with predetermined spaces in the second direction y.

Each of the plurality of ridge portions 12 has a larger amount of continuous fibers than the plurality of groove portions 14 and has a larger thickness than the plurality of groove portions 14. That is, the thickness of the plurality of groove portions 14 is smaller than the thickness of the plurality of ridge portions 12. The thickness of the plurality of ridge portions 12 corresponds to the thickness of the nonwoven fabric 10. The plurality of ridge portions 12 and the plurality of groove portions 14 are connected by a smooth surface. As shown in FIG. 2, for the plurality of ridge portions 12 and the plurality of groove portions 14, with a thickness T₅₀ corresponding to 50% of a thickness Tn of the nonwoven fabric 10 in an unloaded state as a boundary, regions having a thickness of 50% or more of the thickness Tn are defined as the ridge portions 12, and regions having a thickness of less than 50% of the thickness Tn are defined as the plurality of groove portions 14.

Each of the plurality of ridge portions 12 and each of the plurality of groove portions 14 have a plurality of joining portions (not shown) in which intersections of the continuous fibers are thermally joined. Since the plurality of joining portions are not crimped, the plurality of joining portions are not in the form of a film. The nonwoven fabric 10 is bulky since the spaces between the continuous fibers are held, and the shape thereof is easily retained since the intersections are thermally joined. Therefore, the nonwoven fabric 10 easily retains a shape including the plurality of ridge portions 12 and the plurality of groove portions 14.

It is preferable that the fiber orientation of the plurality of ridge portions 12 along the first direction x be higher than the fiber orientation of the plurality of groove portions 14 along the first direction x. Due to the higher fiber orientation of the plurality of ridge portions 12 along the first direction x, the distance between the intersections of the continuous fibers is short, and the distance between the plurality of joining portions is short, which makes it easier to retain the shape. The fiber orientation is a concept consisting of the orientation angle and the orientation intensity of fibers, and can be measured by, for example, the following method. The nonwoven fabric 10 is left to stand so that the surface on which the plurality of ridge portions 12 and the plurality of groove portions 14 are formed is positioned on the upper side. A microscope (for example, a scanning electron microscope such as JCM-5100 manufactured by JEOL Ltd.) is used to take an enlarged image of the nonwoven fabric 10 in a direction perpendicular to the measurement surface of the nonwoven fabric 10, print the enlarged image, and trace the fibers on the transparent PET sheet. The enlarged image is an image enlarged to a magnification capable of measuring 10 or more fibers, and the enlargement magnification is, for example, 50 to 300 times. The image is downloaded into a personal computer and nexusNewQube (standalone version) image processing software manufactured by Nexus Corporation is used to binarize the image. A fiber orientation analysis program, Fiber Orientation Analysis 8.13 Single, is used to obtain the orientation angle and the orientation intensity from the binarized image. The orientation angle is the angle at which the fibers are most oriented, and the orientation intensity is the strength at the orientation angle. The measurement is repeated several times (for example, 3 to 5 times) to calculate the average value.

Each of the plurality of ridge portions 12 has a plurality of recessed portions 16. The recessed portion 16 has an elongated shape having a length in the first direction x that is longer than the length in the second direction y, and a plurality of the recessed portions 16 are arranged with spaces in the first direction x. Each of the plurality of recessed portions 16 is recessed in the thickness direction z, and has a base 18 at the bottom thereof. The base 18 is formed of continuous fibers. The base 18 has a smaller amount of continuous fibers than the portion of each of the plurality of ridge portions 12 excluding the base 18, and has a plurality of joining portions (not shown) in which intersections of the continuous fibers are thermally joined. A thickness Tb of the base 18 is larger than a thickness Tc of each of the plurality of groove portions 14. That is, the thickness Tc of each of the plurality of groove portions 14 is smaller than the thickness Tb of the base 18.

The plurality of ridge portions 12 may have the bundle-shaped portions 20 in one side of at least a part of the recessed portions 16 in the second direction y. The bundle-shaped portion 20 has a plurality of continuous fibers joined at the plurality of joining portions in a state where the plurality of continuous fibers are oriented in the first direction x. The "state where the plurality of continuous fibers are oriented in the first direction x" refers not only to a case where the continuous fibers are oriented parallel to the first direction x, but also to a case where the continuous fibers are oriented in a range in which the angular difference with respect to the first direction x is 30 degrees or less. In the bundle-shaped portion 20, the intersections of the continuous fibers are thermally joined by the plurality of joining portions. The bundle-shaped portion 20 has a smaller inter-fiber distance between the continuous fibers than in a case where the continuous fibers are not oriented in a specific direction. That is, in the bundle-shaped portion 20, since continuous fibers between the joining portions are oriented in the first direction x, the inter-fiber distance between the continuous fibers between the joining portions becomes smaller. Further, in the bundle-shaped portion 20, in a case where a load is applied in the thickness direction z, there is a high probability that the continuous fibers are in line contact with each other, and thus the bundle-shaped portion is less likely to be deformed in the thickness direction z. The bundle-shaped portion 20 may be formed on both sides of the recessed portions 16 in the second direction y.

On the other hand, in a case of continuous fibers that are not oriented in a specific direction, since continuous fibers between the joining portions are oriented in a non-specific direction, when a load is applied in the thickness direction z, the fibers are deformed in a range in which the probability of the fibers coming into point contact with each other is high and the fibers do not come into contact with each other.

The thickness of the nonwoven fabric 10 in the unloaded state, the thickness of the plurality of groove portions 14, and the thickness of the base 18 are measured using a two-dimensional laser displacement meter. As the two-dimensional laser displacement meter, for example, a high precision two-dimensional laser displacement meter LJ-G series (model: LJ-G030) manufactured by Keyence Corporation may be used. The nonwoven fabric is placed on a horizontal measuring table, the displacement from the measuring table for five different portions for each target place of the nonwoven fabric is measured with a laser displacement meter, and the average value of the five measured values is used as the thickness (mm) of each target place of the nonwoven fabric.

Each of the plurality of ridge portions 12 may have a plurality of protrusion portions 22 that protrude toward each of the plurality of adjacent groove portions 14. Each of the plurality of protrusion portions 22 protrudes in the second direction y and is arranged side by side with spaces along the first direction x. A portion between the protrusion portions 22 of each of the plurality of ridge portions 12 is recessed in the second direction y. The plurality of protrusion portions 22 may be located on both sides of the plurality of ridge portions 12, respectively, and the protrusion portions 22 may be located at positions overlapping each other in the second direction y. In a case where the protrusion portions 22 are respectively located at positions overlapping each other in the second direction y, portions having a wide width and portions having a narrow width are respectively formed in the plurality of ridge portions 12 and the plurality of groove portions 14. Further, the protrusion portions 22 may be respectively located at positions not overlapping each other in the second direction y. In a case where the protrusion portions 22 are respectively located at positions not overlapping each other in the second direction y, the plurality of ridge portions 12 and the plurality of groove portions 14 each have a substantially constant width.

Since the thickness Tc of each of the plurality of groove portions 14 is smaller than the thickness Tb of the base 18 due to a small amount of continuous fibers, the overlapping of the continuous fibers in the thickness direction z is reduced. That is, in the plurality of groove portions 14, the space between the continuous fibers becomes larger. Therefore, the nonwoven fabric 10 can be seen from one side to the other side in the thickness direction z through the spaces between the continuous fibers in the plurality of groove portions 14, that is, the nonwoven fabric 10 has excellent visibility.

The visibility evaluation may be performed by using an average transmittance obtained by the following method. First, five samples of 100 mm × 100 mm are prepared. Each sample is arranged on black paper with the protruded and recessed surface facing upward. A 12 megapixel digital camera is used to photograph the entire sample from a position about 15 cm high from the sample so that the entire sample can be photographed. The obtained image is taken into VHX-7000 manufactured by KEYENCE CORPORATION, automatic area measurement is performed, the extraction method is set to brightness (standard), and the image is processed with the following settings to calculate the transmittance. Then, the average value of the transmittances of the five samples is defined as the average transmittance of the nonwoven fabric.
Measurement area: 50 mm × 50 mm
Filter setting: texture removal 10
Brightness: unevenness removal 1
Extraction setting: brightness setting 0 to 148
Filling in blank: OFF
Small particle removal: OFF
Shaping setting: filling in blank Auto

### (Method for Manufacturing Nonwoven Fabric)

The nonwoven fabric 10 according to the above embodiment can be formed by a spunbond method. For example, a resin composition is spun from a spinning nozzle, the spun long fiber filaments are cooled with a cooling fluid or the like, and tension is applied to the long fiber filaments by drawing air to form continuous fibers having a predetermined fineness. The obtained continuous fibers are deposited on the wire mesh while being sucked from the lower side of the wire mesh that moves in the transport direction MD to form a web. To the obtained web, the intersections of the continuous fibers are thermally joined by an air-through method in which hot air is sent from one side of the web. The nonwoven fabric 10 can be manufactured as described above.

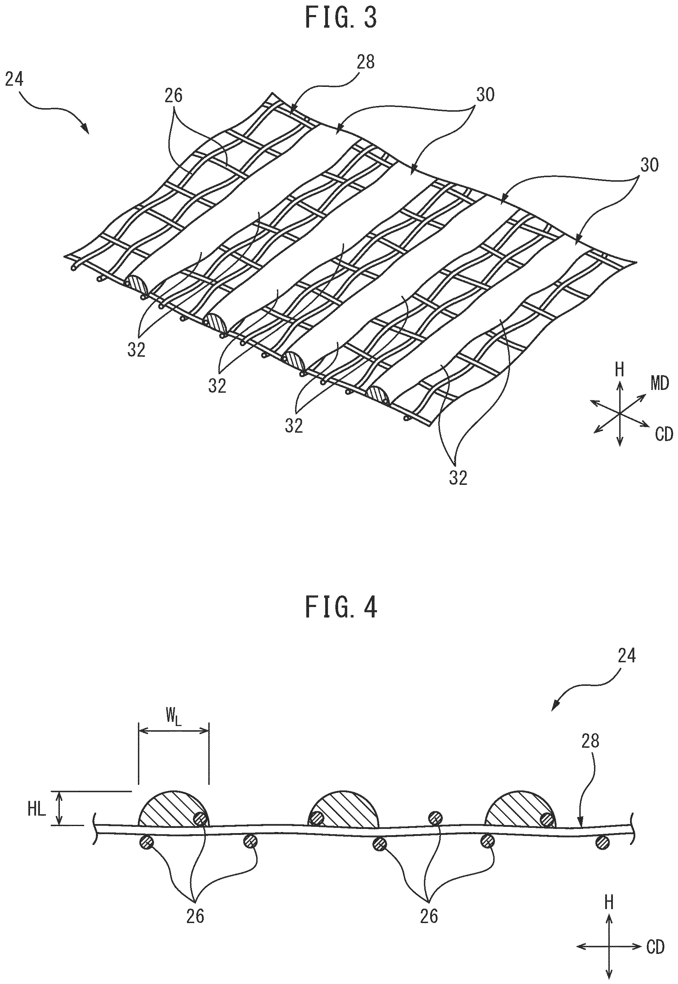

As shown in FIG. 3, a wire mesh 24 has a transport direction MD, a transverse direction CD, and a height direction H that are orthogonal to each other and includes a mesh main body 28 formed of a plurality of wires 26 arranged in a net shape. Each of the plurality of wires 26 has a predetermined outer diameter. The mesh main body 28 may be formed by arranging the plurality of wires 26 in vertical lines and horizontal lines and intersecting the wires one by one while holding a certain space.

The wire mesh 24 has a plurality of ribs 30 that protrude in the height direction H from one surface of the mesh main body 28 in the height direction H and extend along the transport direction MD. The ribs 30 are arranged with predetermined spaces in the transverse direction CD. The ribs 30 block a part of the gaps in the mesh main body 28 arranged along the first direction x between the wires 26.

As shown in FIG. 4, a height H_{L} of the rib 30 may be 1.0 mm or more. It is preferable that the height H_{L} of the rib 30 be larger than the outer diameter of the wire 26, that is, the outer diameter of the wire 26 be smaller than the height H_{L} of the rib 30. A width W_{L} of each of the plurality of ribs 30, that is, the total length of the lengths of the ribs 30 in the transverse direction CD may be 50% or less of the length of the mesh main body 28 in the transverse direction CD. The width W_{L} of each of the ribs 30 is not limited to a case where the width of each of the ribs is constant, and includes a case where the width of each of the ribs is partially different. The width W_{L} of each of the ribs 30 may be the average value of values measured at a plurality of places (for example, five places) with predetermined spaces (for example, 100 mm) in the transport direction MD. The width W_{L} of the rib 30 is defined as the length of the place in contact with the mesh main body 28 in the transverse direction CD.

Each of the plurality of ribs 30 may have a plurality of bulging portions 32 that bulge in the transverse direction CD. The bulging portions 32 are arranged side by side with predetermined spaces along the transport direction MD. A portion between the bulging portions 32 of each of the plurality of ribs 30 is recessed in the transverse direction CD. The plurality of bulging portions 32 may be respectively located on both sides of the ribs 30, and the bulging portions 32 may be respectively located at positions overlapping each other in the transverse direction CD. In a case where the bulging portions 32 are respectively located at positions overlapping each other in the transverse direction CD, a wide portion and a narrow portion are respectively formed in the rib 30 and between the ribs 30. Further, the bulging portions 32 may be respectively located at positions not overlapping each other in the transverse direction CD. In a case where the bulging portions 32 are respectively located at positions not overlapping each other in the transverse direction CD, a portion having a constant width is formed in the rib 30 and between the ribs 30.

Using the above-described wire mesh 24, continuous fibers
are deposited on the wire mesh 24 by being sucked from the lower side of the wire mesh 24. Some of the continuous fibers are preferentially collected between the ribs 30 in which the ribs 30 of the mesh main body 28 are not formed. When some of the continuous fibers are preferentially collected between the ribs 30, the continuous fibers become dense and the plurality of ridge portions 12 having a high basis weight are formed. Further, on the ribs 30, the continuous fibers become sparse, and a plurality of groove portions 14 having a low basis weight are formed. Further, in a state of being deposited on the wire mesh 24, the continuous fibers are thermally joined, and thereby the shapes of the plurality of ridge portions 12 and the plurality of groove portions 14 are maintained. The nonwoven fabric 10 shown in FIG. 1 is obtained by removing the nonwoven fabric from the wire mesh 24 and turning the nonwoven fabric upside down in the thickness direction z. Therefore, in the method of manufacturing the nonwoven fabric 10, the nonwoven fabric 10 formed of continuous fibers, including the plurality of ridge portions 12 and the plurality of groove portions 14, and having the plurality of joining portions in which intersections of the continuous fibers are thermally joined can be manufactured.

Since the height of the rib 30 is 1.0 mm or more, the continuous fibers are less likely to remain on the rib 30, and the continuous fibers are accumulated between the ribs 30. Therefore, the plurality of ridge portions 12 having a predetermined thickness can be formed. Therefore, in the method of manufacturing the nonwoven fabric 10, a nonwoven fabric 10 having ridge portions 12 and groove portions 14 can be more reliably manufactured. Since the continuous fibers are accumulated between the ribs 30 along the ribs 30 so as to avoid the ribs 30, the fiber orientation of the plurality of ridge portions 12 along the first direction x becomes higher than the fiber orientation of the plurality of groove portions 14 along the first direction x.

Since the total length of the lengths of each of the plurality of ribs 30 in the transverse direction CD is 50% or less of the length of the mesh main body 28 in the transverse direction CD, a sufficient amount of airflow of gas passes through the mesh main body 28 excluding the ribs 30. Therefore, in the method of manufacturing the nonwoven fabric 10, since the continuous fibers can be stably deposited on the wire mesh 24, a nonwoven fabric 10 having a plurality of ridge portions 12 and a plurality of groove portions 14 can be more reliably manufactured.

Since each of the plurality of ribs 30 has a plurality of bulging portions 32 that bulge in the transverse direction CD, the continuous fibers are sparse in the bulging portions 32. The continuous fibers become dense between the bulging portions 32 in the first direction x, and the portions between the bulging portions 32 are protrusion portions 22 protruding from the plurality of ridge portions 12 toward the plurality of groove portions 14. Therefore, in the above-described manufacturing method, the nonwoven fabric 10 having the protrusion portions 22 in the plurality of ridge portions 12 can be manufactured.

Since the outer diameter of the wire 26 that forms the wire mesh 24 is smaller than the height of the rib 30, the rib 30 is higher than the wire 26 even in the portion in which the wires 26 of the wire mesh 24 overlap each other, and thus the plurality of groove portions 14 are formed. Therefore, in the method of manufacturing the nonwoven fabric 10, the nonwoven fabric 10 having the plurality of ridge portions 12 and the plurality of groove portions 14 can be more reliably manufactured. Further, since the portion in which the wires 26 of the wire mesh 24 overlap each other is smaller than the rib 30, the continuous fibers are less likely to be deposited in the portion in which the wires 26 of the wire mesh 24 overlap each other and are collected in the mesh. Thus, the plurality of ridge portions 12 having the recessed portions 16 can be formed.

Further, when the continuous fibers are accumulated between the ribs 30 on the wire mesh 24, the continuous fibers are collected in the mesh while avoiding a portion which protrudes upward in the height direction in the portion in which the wires 26 overlap each other, and thus the plurality of continuous fibers are brought into a state of being oriented in the first direction x. When the joining portion is formed in this state, a bundle-shaped portion in which the plurality of continuous fibers are oriented in the transport direction MD, that is, in the first direction x is formed.

It is preferable that the continuous fibers be deposited on the wire mesh 24 at a fiber speed of 1000 m/min or more and 4500 m/min or less. Since the continuous fibers have a fiber speed of 1000 m/min or more, the continuous fibers are easily deposited on the wire mesh 24, and the plurality of ridge portions 12 are easily formed. Further, when the fiber speed of the continuous fiber is 4500 m/min or less, the orientation of the continuous fibers can be prevented from being disturbed on the wire mesh 24, and the plurality of ridge portions 12 are easily formed. Therefore, when the number of continuous fibers is within the above range, the nonwoven fabric 10 having the plurality of ridge portions 12 and the plurality of groove portions 14 can be efficiently manufactured.

### (Examples of Absorbent Article)

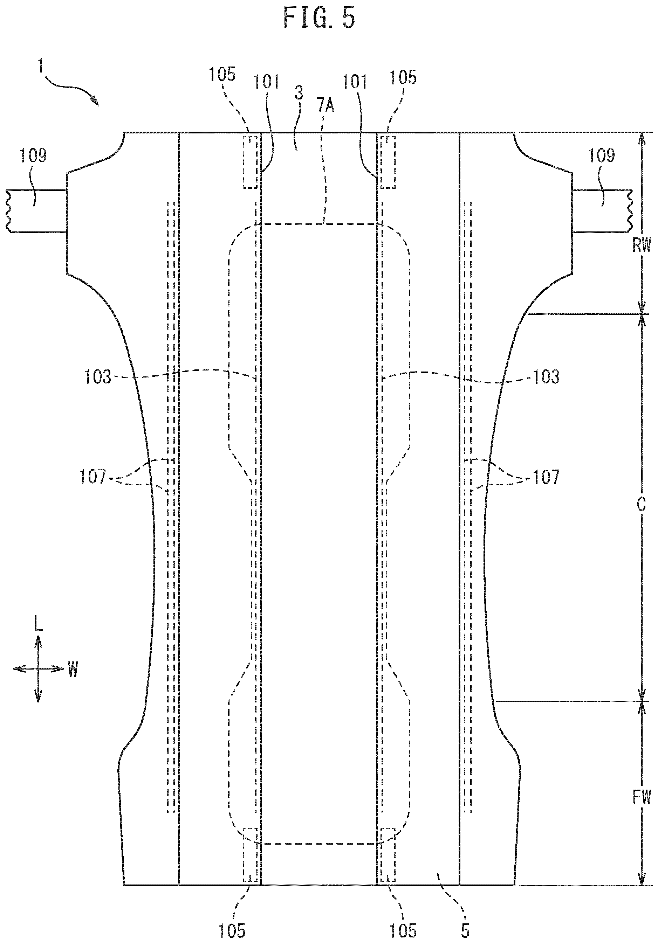

An absorbent article 1 shown in FIG. 5 is an example of a disposable diaper and includes a plurality of layers. The absorbent article 1 includes a liquid-permeable sheet 3 as a skin contacting sheet that comes into contact with the skin of a wearer, a liquid-impermeable sheet 5, and an absorbent body 7 that is arranged between the liquid-permeable sheet 3 and the liquid-impermeable sheet 5. The absorbent article 1 may further include an exterior sheet (not shown) on the non-skin side of the liquid-impermeable sheet 5. In addition, the absorbent article may have an indicator (not shown) and a pattern (not shown). The indicator is preferably arranged between the absorbent body 7 and the liquid-impermeable sheet 5. The pattern is preferably arranged on the inner surface or the outer surface of the liquid-impermeable sheet 5. In a lengthwise direction L, the absorbent article 1 is divided into three regions: a front waist region FW, a rear waist region RW, and a crotch region C between the front waist region FW and the rear waist region RW. The absorbent article 1 further includes a pair of leakproof walls 101 including an elastic member 103, a fixed portion 105 that fixes the leakproof wall 101 to the liquid-permeable sheet 3, an elastic member 107 arranged around the leg portion, a tape fastener 109, and the like. It should be noted that these are well-known in the art and will not be described here.

The liquid-permeable sheet 3 may be the nonwoven fabric 10. In a case where the nonwoven fabric is applied to the liquid-permeable sheet 3, the nonwoven fabric 10 is liquid-permeable. The liquid-permeable sheet 3 is arranged such that a surface on which the plurality of ridge portions 12 and the plurality of groove portions 14 of the nonwoven fabric 10 are located is arranged on the skin side. The liquid-permeable sheet 3 may be arranged such that the first direction x of the nonwoven fabric 10 extends along the lengthwise direction L of the absorbent article 1. In the liquid-permeable sheet 3, since the plurality of groove portions 14 and the plurality of ridge portions 12 are arranged along the lengthwise direction L of the absorbent article 1, the bodily fluid is easily diffused in the lengthwise direction L of the absorbent article 1.

Further, the liquid-permeable sheet 3 may be arranged such that the first direction x of the nonwoven fabric 10 extends along the width direction W of the absorbent article 1. In the liquid-permeable sheet 3, since the plurality of groove portions 14 and the plurality of ridge portions 12 are arranged along the width direction W of the absorbent article 1, the bodily fluid is easily diffused in the width direction W of the absorbent article 1.

The exterior sheet may be the nonwoven fabric 10. The exterior sheet may have a surface on which the plurality of ridge portions 12 and the plurality of groove portions 14 of the nonwoven fabric 10 are located on the non-skin side, or may have the surface on the skin side. The exterior sheet may be arranged such that the first direction x of the nonwoven fabric 10 extends along the lengthwise direction L of the absorbent article 1 or extends along the width direction W.

Since the nonwoven fabric 10 has little change in thickness, the shapes of the plurality of ridge portions 12 and the plurality of groove portions 14 are easily retained. In the nonwoven fabric 10, in a case where a load is applied in the thickness direction z after the absorbent article is put on, the plurality of ridge portions 12 are less likely to become thin, and the length of the plurality of groove portions 14 in the second direction y is maintained in a state where the basis weight is small.

The absorbent article is not limited to a disposable diaper, and can be applied to, for example, a urine absorbing pad, a urination sheet for animals, and the like, an absorbent article mainly absorbing menstrual blood and the like, such as a sanitary napkin, a panty liner, and the like.

### (Action and Effect)

The nonwoven fabric 10 is formed of continuous fibers, and each of the plurality of ridge portions 12 and each of the plurality of groove portions 14 has the plurality of joining portions in which intersections of the continuous fibers are thermally joined. The nonwoven fabric 10 easily retains its shape, and the change in thickness of the nonwoven fabric 10 in a state where a load is applied in the thickness direction z is small compared with the nonwoven fabric 10 formed of short fibers. For example, when the thickness of the nonwoven fabric 10 when receiving a pressure of 0.05 kPa (0.5 gf/cm²) is denoted as T0, and the thickness of the nonwoven fabric 10 when receiving a pressure of 4.9 kPa (50 gf/cm²) is denoted as Tm, the compression characteristics (Tm/T0) of the nonwoven fabric 10 are more than 0.3 or 0.4 or more. Therefore, the nonwoven fabric 10 is less likely to become thin, and for example, in a case where the nonwoven fabric is applied to the liquid-permeable sheet 3 of the disposable diaper, there is little change in thickness while the diaper is put on. Thus, a state where a space between the absorbent body 7 and the skin of the user is retained is easily maintained. Due to that, the nonwoven fabric 10 is excellent in suppressing a wet feeling.

The thickness T0 and the thickness Tm are measured using an automated compression tester, KES-FB3-A, manufactured by KATO TECH CO., LTD. The measurement conditions are as follows.
SENS: 2
Speed: 0.02 mm/sec
Stroke: 5 mm/10 V
Compression area: 2 cm²
Uptake interval: 0.1 second
Upper limit load: 50 g/cm²
Number of repetitions: 1

Since the plurality of groove portions 14 have a lower basis weight than the plurality of ridge portions 12 and have the plurality of joining portions in which intersections of the continuous fibers are thermally joined, the shape of the plurality of groove portions 14 is easily retained. Accordingly, in a case where a load is applied in the thickness direction z, the thickness of the ridge portions 12 is less likely to be changed. In such a case, since the length of the ridge portion 12 in the second direction y is less likely to be changed, the length of the groove portion 14 in the second direction y is less likely to be changed. That is, since the structures of the ridge portions 12 and the groove portions 14 are less likely to be changed, and the length of the groove portions 14 in the second direction y is less likely to be changed in the nonwoven fabric 10, the range in which visibility can be obtained is less likely to be changed. Therefore, the plurality of groove portions 14 have excellent visibility in the thickness direction z through the plurality of groove portions 14. Since the nonwoven fabric 10 has the plurality of groove portions 14, for example, in a case where the nonwoven fabric 10 is applied to the liquid-permeable sheet 3 of the disposable diaper, the visibility for the bodily fluid absorbed by the absorbent body 7 is excellent.

Since the plurality of ridge portions 12 and the plurality of groove portions 14 are alternately located in the second direction y, the nonwoven fabric 10 is excellent in suppressing a wet feeling by retaining a space between the absorbent body 7 and the skin of the user in a wide range in the first direction x and the second direction y, and has excellent the visibility for the bodily fluid absorbed by the absorbent body 7 through the plurality of groove portions 14.

In the nonwoven fabric 10, in a case where a load is applied in the thickness direction z after the absorbent article is put on, the plurality of ridge portions 12 are less likely to collapse, and the length of the plurality of groove portions 14 in the second direction y is maintained in a state where the basis weight is small. For example, in a case where the nonwoven fabric 10 is applied to the exterior sheet of the disposable diaper and the surface on which the plurality of ridge portions 12 and the plurality of groove portions 14 are located is arranged on the non-skin side, even in a case where the nonwoven fabric 10 receives a load over time after the absorbent article is put on, excellent breathability and visibility in the plurality of groove portions 14 are maintained. Therefore, in the absorbent article 1, excellent breathability is obtained after the absorbent article 1 is put on, and the indicator or the pattern located on the liquid-impermeable sheet 5 can be satisfactorily visually recognized from the exterior sheet side. In addition, in a case where the nonwoven fabric 10 is applied to the exterior sheet and the surface on which the plurality of ridge portions 12 and the plurality of groove portions 14 are located is arranged on the skin side, a space is formed between the nonwoven fabric 10 and the liquid-impermeable sheet 5, which makes it difficult to transmit moisture to clothing, sheets, or the like.

In the nonwoven fabric 10, since the nonwoven fabric 10 has the plurality of groove portions 14 and the plurality of recessed portions 16 recessed in the thickness direction z in the plurality of ridge portions 12, the area where the skin of the user touches the wet nonwoven fabric 10 is small compared with a nonwoven fabric 10 having no recessed portions 16. In the nonwoven fabric 10, for example, in a case where the nonwoven fabric 10 is applied to the liquid-permeable sheet 3 of the disposable diaper, the base 18 absorbs the excreted bodily fluid and more reliably prevents the bodily fluid absorbed by the absorbent body 7 from returning into the recessed portions 16. Therefore, the nonwoven fabric 10 is excellent in suppressing a wet feeling. In addition, in the nonwoven fabric 10, for example, in a case where the nonwoven fabric 10 is applied to the exterior sheet of the disposable diaper, the surface on which the plurality of ridge portions 12 and the plurality of groove portions 14 are located may be arranged on the non-skin side. In this case, since the contact area of the surface of the nonwoven fabric 10 is decreased by the amount corresponding to the presence of the recessed portions 16, the nonwoven fabric 10 can give a soft tactile feel and has a fabric-like complex surface shape. Further, in the nonwoven fabric 10, the surface on which the plurality of ridge portions 12 and the plurality of groove portions 14 are located may be arranged on the skin side. In this case, the space formed between the nonwoven fabric 10 and the liquid-impermeable sheet 5 is further increased by the amount corresponding to the recessed portions, which makes it difficult to transmit moisture to clothing, sheets, or the like.

In the nonwoven fabric 10, each of the plurality of ridge portions 12 has a bundle-shaped portion 20 joined at the plurality of joining portions in a state where the continuous fibers are oriented along the first direction x on one side of at least a part of the plurality of recessed portions 16 in the second direction y. The bundle-shaped portion 20 has a small inter-fiber distance between the continuous fibers and is less likely to be deformed when receiving a load. Therefore, the nonwoven fabric 10 having the bundle-shaped portion 20 easily retains the shape of the recessed portions in which there is little change in thickness when the absorbent article is put on. In the nonwoven fabric 10, for example, in a case where the nonwoven fabric 10 is applied to the liquid-permeable sheet 3 of the disposable diaper, a state where a space between the absorbent body 7 and the skin of the user is retained is easily maintained, and the shape of the recessed portions 16 is easily retained. Thus, a state where the area where the skin of the user touches the nonwoven fabric 10 is small is easily maintained.

In the nonwoven fabric 10, since the thickness of each of the plurality of groove portions 14 is smaller than the thickness of the base 18, for example, in a case where the nonwoven fabric 10 is applied to the liquid-permeable sheet 3 or the exterior sheet of the disposable diaper, the plurality of groove portions 14 have excellent visibility in the thickness direction z. Therefore, the difference from the plurality of ridge portions 12 where almost no visibility in the thickness direction z is obtained is remarkable. Therefore, the nonwoven fabric 10 has excellent visibility. Further, since the nonwoven fabric 10 is formed of continuous fibers and has an average breaking strength per predetermined average basis weight, the nonwoven fabric 10 can be applied to the absorbent article 1 as the liquid-permeable sheet 3 and the exterior sheet having excellent visibility.

Since the continuous fibers are conjugated fibers containing an olefin-based resin, the joining portions can be formed even at a low heat treatment temperature, and thus the joining portions can be more efficiently formed in the entire nonwoven fabric 10. Since the joining portions are formed in the entire nonwoven fabric 10, even in a case where a load is applied in the thickness direction z after the absorbent article is put on, the shape is easily retained, which makes it difficult to change the structures of the ridge portions 12 and the groove portions 14. In the nonwoven fabric 10 in which the joining portions are formed throughout, the shape of the groove portions 14 is retained, which makes it possible to enhance visibility and to suppress the deformation of the plurality of groove portions 14 and the plurality of ridge portions 12 due to excrement. Therefore, the nonwoven fabric 10 is excellent in suppressing a wet feeling.

In the nonwoven fabric 10, the average fiber diameter of the continuous fibers is 15 µm or more and 30 µm or less, and the average basis weight of the nonwoven fabric 10 is 10 g/m² or more and 40 g/m² or less. Therefore, when a load is applied in the thickness direction z after the absorbent article is put on, the structures of the ridge portions 12 and the groove portions 14 that easily retain the shape can be more reliably obtained. Further, after the excreted bodily fluid is absorbed, the continuous fibers can maintain the space between the continuous fibers. Therefore, for example, in a case where the nonwoven fabric 10 is applied to the liquid-permeable sheet 3 of the disposable diaper, the nonwoven fabric 10 is excellent in suppressing a wet feeling by retaining a space between the absorbent body 7 and the skin of the user, and has excellent the visibility for the bodily fluid absorbed by the absorbent body 7.

In the nonwoven fabric 10, since the plurality of groove portions 14 and the plurality of ridge portions 12 are arranged along the lengthwise direction L of the absorbent article 1, the nonwoven fabric 10 has excellent visibility in the lengthwise direction L. In addition, in the nonwoven fabric 10, the bodily fluid is easily diffused in the lengthwise direction L of the absorbent article 1. Therefore, in a case where the nonwoven fabric 10 is applied to the liquid-permeable sheet 3, the nonwoven fabric 10 prevents the bodily fluid from staying at one place by diffusing the bodily fluid in the lengthwise direction L, and thus the nonwoven fabric 10 is excellent in suppressing a wet feeling.

In the nonwoven fabric 10, since the plurality of groove portions 14 and the plurality of ridge portions 12 are arranged along the width direction W of the absorbent article 1, the nonwoven fabric 10 has excellent visibility in the width direction W. In addition, in the nonwoven fabric 10, the bodily fluid is easily diffused in the width direction W of the absorbent article 1. Therefore, in a case where the nonwoven fabric 10 is applied to the liquid-permeable sheet 3, the nonwoven fabric 10 prevents the bodily fluid from staying at one place by diffusing the bodily fluid in the width direction W, and thus the nonwoven fabric 10 is excellent in suppressing a wet feeling.

In the nonwoven fabric 10, since each of the plurality of ridge portions 12 has the plurality of protrusion portions 22 that protrude toward each of the plurality of adjacent groove portions 14 and are arranged side by side with spaces along the first direction x, the plurality of ridge portions 12 are less likely to collapse in a case where a load is applied in the thickness direction z. Therefore, for example, in a case where the nonwoven fabric 10 is applied to the liquid-permeable sheet 3 or the exterior sheet of the disposable diaper, a state where the shape of the plurality of ridge portions 12 is retained is easily maintained, the plurality of ridge portions 12 do not block the groove portions 14, and thus visibility is excellent.

In the nonwoven fabric 10, since the fiber orientation of the plurality of ridge portions 12 along the first direction x is higher than the fiber orientation of the plurality of groove portions 14 along the first direction x, a distance between the intersections of the continuous fibers is short, and a distance between the plurality of joining portions is short. Therefore, the nonwoven fabric 10 easily retains the shape of the plurality of ridge portions 12. Further, the bodily fluid can be diffused along the plurality of ridge portions 12 in the first direction x. Therefore, since the nonwoven fabric 10 prevents the bodily fluid from staying at one place by diffusing the bodily fluid in the first direction x, the nonwoven fabric 10 is excellent in suppressing a wet feeling. In addition, in the nonwoven fabric 10, due to the higher fiber orientation of the plurality of ridge portions 12 along the first direction x, the distance between the intersections of the continuous fibers is short, and the distance between the plurality of joining portions is short, which makes it easier to retain the shape. Therefore, the nonwoven fabric 10 is less likely to be deformed in the thickness direction z.

Since the nonwoven fabric 10 is a spunbond nonwoven fabric, in a case where the continuous fibers are moved by an airflow, the continuous fibers are moved across a certain length due to the continuous fibers having a long fiber length, and further moved along with adjacent continuous fibers. Therefore, the continuous fibers are easily affected by the airflow in the deposition step. Accordingly, when each of the continuous fibers is moved, a portion having a small inter-fiber distance is easily formed. In the continuous fibers, the distance between the intersections of the continuous fibers is short, and the distance between the plurality of joining portions is short. Therefore, the nonwoven fabric 10 easily retains the shape of the plurality of ridge portions 12.

The nonwoven fabric 10 is less likely to be deformed when receiving a load, and there is little change in thickness even after the load is removed. The change in thickness before and after the application of the load is measured as follows.

First, a take-up roll of the nonwoven fabric 10 is prepared. The nonwoven fabric 10 is cut out from the take-up roll. The thickness of the nonwoven fabric 10 when receiving a load of 0.3 kPa (3 gf/cm²) immediately after cutting out is denoted as T1. The cut nonwoven fabric 10 is allowed to stand for 1 day in an environment at a temperature of 23°C and a humidity of 60%. Then, the thickness of the nonwoven fabric 10 when receiving the load of 0.3 kPa (3 gf/cm²) is denoted as T2. The thickness recoverability (T2/T1) of the nonwoven fabric 10 is less than 1.1, and there is almost no change in thickness. Therefore, since the change in thickness is small even after the load is removed, the upper side and the lower side of the nonwoven fabric 10 in the thickness direction z are less likely to be displaced in the planar direction, and good visibility is maintained.

### Examples

The nonwoven fabrics corresponding to the above embodiments are actually prepared and evaluated. Hereinafter, the present invention will be described by showing examples, but the present invention is not limited to these examples.

### (1) Sample

### [Examples 1 to 3]

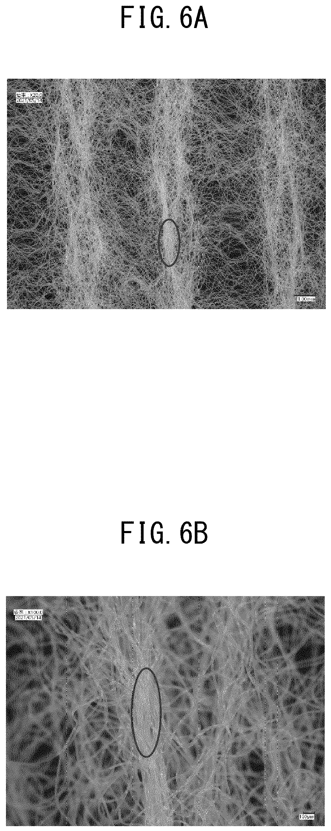

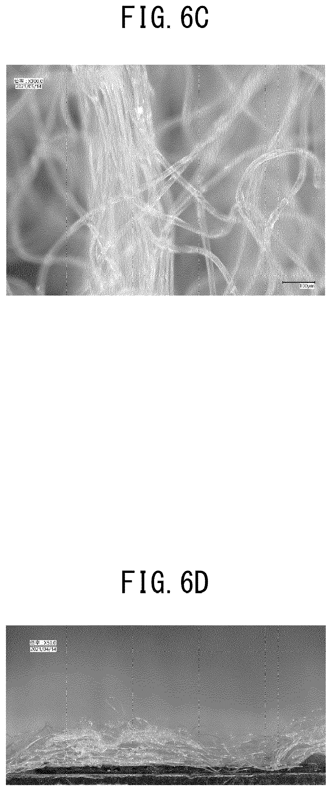

According to the description in the "Method for Manufacturing Nonwoven Fabric" above, nonwoven fabrics of Examples 1 to 3 each of which is formed of continuous fibers are prepared by a spunbond method. All of the nonwoven fabrics of Examples 1 to 3 have a plurality of ridge portions, a plurality of groove portions, a plurality of recessed portions, and a plurality of protrusion portions. The photographs of the nonwoven fabric of Example 1 are shown in FIGS. 6A, 6B, 6C and 6D. An ellipse is shown in FIGS. 6A and 6B, and the ellipse represents a specific portion and does not constitute the nonwoven fabric. As shown in FIGS. 6A and 6B, the plurality of recessed portions are formed in the plurality of ridge portions, and a bundle-shaped portion is formed adjacent to the recessed portion. As shown in FIG. 6C, in the bundle-shaped portion, the plurality of continuous fibers are oriented along the first direction and joined at the plurality of joining portions. As shown in FIG. 6D, the thickness of the plurality of groove portions is smaller than the thickness of the base.

### Comparative Example 1 & 2

Nonwoven fabrics of Comparative Examples 1 and 2 each of which is formed of short fibers are prepared by a carding method. In the nonwoven fabric of Comparative Example 1, high basis weight portions and low basis weight portions are formed according to the method described in Patent Literature 1. In the nonwoven fabric of Comparative Example 2, a plurality of groove portions are formed by hot embossing. The plurality of groove portions and the ridge portions between the plurality of groove portions of Comparative Example 2 have the same basis weight. The nonwoven fabrics of Comparative Example 1 and Comparative Example 2 have no recessed portions and no protrusion portions.

### Evaluation results

The characteristics of the nonwoven fabrics of Examples and Comparative Examples are as shown in Table 1.

For the thickness T1 when the nonwoven fabric receives a load of 0.3 kPa (3 gf/cm²), the thicknesses of the nonwoven fabrics in Examples 1 to 3 are larger than the thicknesses in Comparative Examples 1 and 2 in which the average basis weights are approximately the same. The thickness recoverability (T2/T1) of all of Examples 1 to 3 is 1.0, while the thickness recoverability (T2/T1) of Comparative Examples 1 and 2 is 1.1 or more. It is confirmed that the nonwoven fabrics of Examples have little change in thickness before and after the application of the load. In the nonwoven fabrics of Examples, since there is little change in the thickness before and after the application of the load, the upper side and the lower side of the nonwoven fabric in the thickness direction are less likely to be displaced in the planar direction, and good visibility is easily maintained.

The compression characteristics (Tm/T0) of Examples 1 to 3 are 0.4 or more, whereas the compression characteristics (Tm/T0) of Comparative Examples 1 and 2 are 0.3. In the nonwoven fabrics of Examples, a change in thickness in a state where a load is applied in the thickness direction is small compared with a nonwoven fabric formed of short fibers. Therefore, the nonwoven fabrics of Examples are less likely to become thin, and in a case where the nonwoven fabric is applied to the liquid-permeable sheet of the disposable diaper, there is little change in thickness while the diaper is put on. Thus, a state where a space between the absorbent body and the skin of the user is retained is easily maintained, and the nonwoven fabrics are excellent in suppressing a wet feeling.

The average transmittance of the nonwoven fabric of each of Examples is excellent compared with Comparative Examples. Since the nonwoven fabrics of Examples have the plurality of joining portions in which intersections of the continuous fibers are thermally joined and the shape is easily retained, the length of the groove portions in the second direction is easily retained. Therefore, the average transmittance of the nonwoven fabrics is high, that is, a state in which visibility is excellent is easily maintained.

**[Table 1]**

| | Examples | | | Comparative Example | |
|---|---|---|---|---|---|
| | 1 | 2 | 3 | 1 | 2 |
| Type of fiber (sheath/core) | PE/PP | PE/PP | PE/PP | PE/PET | PE/PET |
| Average fiber diameter (µm) | 18.63 | 19.18 | 19.21 | 16.14 | 17.08 |
| Average basis weight (g/m²) | 25.41 | 21.30 | 16.79 | 22.65 | 27.20 |
| Thickness T1 (3 gf/cm²) (mm) | 0.45 | 0.34 | 0.33 | 0.24 | 0.26 |
| Thickness T2 (0 gf/cm²) (mm) | 0.45 | 0.35 | 0.32 | 0.29 | 0.28 |
| Thickness recoverability (T2/T1) | 1.0 | 1.0 | 1.0 | 1.2 | 1.1 |
| Thickness T0 (0.5 gf/cm²) (mm) | 0.60 | 0.39 | 0.37 | 0.38 | 0.41 |
| Thickness Tm (50 gf/cm²) (mm) | 0.29 | 0.16 | 0.16 | 0.10 | 0.13 |
| Compression characteristics (Tm/T0) | 0.5 | 0.4 | 0.4 | 0.3 | 0.3 |
| Average transmittance of nonwoven fabric (50 × 50 mm) (%) | 56.64 | 58.14 | 59.51 | 24.12 | 15.59 |

### REFERENCE SIGNS LIST

1: absorbent article
3: liquid-permeable sheet
7: absorbent body
10: nonwoven fabric
12: ridge portion
16: recessed portion
18: Base portion
20: bundle-shaped portion
22: protrusion portion
24: wire mesh
26: wire
28: mesh main body
30: rib
32: bulging portion

## Claims

1. A nonwoven fabric for an absorbent article, having a first direction (x), a second direction (y), and a thickness direction (z) that are orthogonal to each other and formed of continuous fibers, the nonwoven fabric comprising:
a plurality of ridge portions (12) that extend along the first direction; and
a plurality of groove portions (14) that extend along the first direction and have a basis weight lower than that of the plurality of ridge portions, wherein
each of the plurality of ridge portions and each of the plurality of groove portions are alternately located in the second direction, and
each of the plurality of ridge portions and each of the plurality of groove portions have a plurality of joining portions in which intersections of the continuous fibers are thermally joined, **characterised in that**
each of the plurality of ridge portions has a plurality of recessed portions (16) recessed in the thickness direction, and a base (18) formed of the continuous fibers at a bottom of each of the plurality of recessed portions.

2. The nonwoven fabric according to Claim 1, wherein
the nonwoven fabric is for a liquid-permeable sheet of the absorbent article.

3. The nonwoven fabric according to Claim 1, wherein
the nonwoven fabric is for an exterior sheet of the absorbent article.

4. The nonwoven fabric according to Claim 1, wherein
each of the plurality of ridge portions has a bundle-shaped portion (20) joined at the plurality of joining portions in a state where the continuous fibers are oriented along the first direction on one side of at least a part of the plurality of recessed portions in the second direction.

5. The nonwoven fabric according to Claim 1 or 4, wherein
a thickness of each of the plurality of groove portions is smaller than a thickness of the base.

6. The nonwoven fabric according to any one of Claims 1 to 5, wherein
the continuous fibers are conjugated fibers containing an olefin-based resin.

7. The nonwoven fabric according to any one of Claims 1 to 6, wherein
an average fiber diameter of the continuous fibers is 15 µm or more and 30 µm or less, and an average basis weight of the nonwoven fabric is 10 g/m² or more and 40 g/m² or less.

8. The nonwoven fabric according to any one of Claims 1 to 7, wherein
the first direction is arranged in a lengthwise direction of the absorbent article.

9. The nonwoven fabric according to any one of Claims 1 to 7, wherein
the first direction is arranged in a width direction of the absorbent article.

10. The nonwoven fabric according to any one of Claims 1 to 9, wherein
each of the plurality of ridge portions has a plurality of protrusion portions (22) that protrude toward each of the plurality of adjacent groove portions and are arranged side by side with spaces along the first direction.

11. The nonwoven fabric according to any one of Claims 1 to 10, wherein
fiber orientation of each of the plurality of ridge portions along the first direction is higher than fiber orientation of each of the plurality of groove portions along the first direction.

12. The nonwoven fabric according to any one of Claims 1 to 11, wherein
the nonwoven fabric is a spunbond nonwoven fabric.

13. A method of manufacturing the nonwoven fabric according to any one of Claims 1 to 12, the method comprising:
a step of, while sucking the continuous fibers from a lower side of a wire mesh having a transport direction, a transverse direction, and a height direction that are orthogonal to each other, depositing the continuous fibers on the wire mesh; and
a step of thermally joining the continuous fibers deposited on the wire mesh, wherein
the wire mesh includes
a mesh main body (28), and
a plurality of ribs (30)that extend along the transport direction, are arranged on the mesh main body with predetermined spaces in the transverse direction, and protrude toward the height direction.

14. The method of manufacturing the nonwoven fabric according to Claim 13, wherein
a height of the rib is 1.0 mm or more.

15. The method of manufacturing the nonwoven fabric according to Claim 13 or 14, wherein
a total length of lengths of each of the plurality of ribs in the transverse direction is 50% or less of a length of the mesh main body in the transverse direction.

16. The method of manufacturing the nonwoven fabric according to any one of Claims 13 to 15 wherein
each of the plurality of ribs has a plurality of bulging portions that bulge in the transverse direction.

17. The method of manufacturing the nonwoven fabric according to any one of Claims 13 to 16wherein
an outer diameter of a wire that forms the wire mesh is smaller than a height of the rib.

18. The method of manufacturing the nonwoven fabric according to any one of Claims 13 to 17, wherein
the continuous fibers are deposited on the wire mesh at a fiber speed of 1000 m/min or more and 4500 m/min or less.

## Patentansprüche

1. Vliesstoff für einen absorbierenden Artikel, der eine erste Richtung (x), eine zweite Richtung (y) und eine Dickenrichtung (z), die rechtwinklig zueinander vorliegen, aufweist und aus kontinuierlichen Fasern ausgebildet ist, wobei der Vliesstoff Folgendes umfasst:
eine Vielzahl von Rippenteilen (12), die sich entlang der ersten Richtung erstrecken; und
eine Vielzahl von Rillenteilen (14), die sich entlang der ersten Richtung erstrecken und ein Flächengewicht aufweisen, das geringer ist als das der Vielzahl von Rippenteilen, wobei
sich jeder der Vielzahl von Rippenteilen und jeder der Vielzahl von Rillenteilen abwechselnd in der zweiten Richtung befinden und
jeder der Vielzahl von Rippenteilen und jeder der Vielzahl von Rillenteilen eine Vielzahl von Verbindungsteilen aufweisen, in denen Schnittstellen der kontinuierlichen Fasern thermisch verbunden sind, **dadurch gekennzeichnet, dass**
jeder der Vielzahl von Rippenteilen Folgendes aufweist: eine Vielzahl von vertieften Teilen (16), die in der Dickenrichtung vertieft sind, und eine Basis (18), die aus den kontinuierlichen Fasern an einer Unterseite von jedem der Vielzahl von vertieften Teilen ausgebildet ist.

2. Vliesstoff nach Anspruch 1, wobei
der Vliesstoff für eine flüssigkeitsdurchlässige Lage des absorbierenden Artikels vorgesehen ist.

3. Vliesstoff nach Anspruch 1, wobei
der Vliesstoff für eine äußere Lage des absorbierenden Artikels vorgesehen ist.

4. Vliesstoff nach Anspruch 1, wobei
jeder der Vielzahl von Rippenteilen einen bündelförmigen Teil (20) aufweist, der an der Vielzahl von Verbindungsteilen in einem Zustand verbunden ist, wo die kontinuierlichen Fasern entlang der ersten Richtung auf einer Seite von mindestens einem Teil der Vielzahl von vertieften Teilen in der zweiten Richtung ausgerichtet sind.

5. Vliesstoff nach Anspruch 1 oder 4, wobei
eine Dicke von jedem der Vielzahl von Rillenteilen kleiner ist als eine Dicke der Basis.

6. Vliesstoff nach einem der Ansprüche 1 bis 5, wobei
die kontinuierlichen Fasern konjugierte Fasern sind, die ein Olefin-basiertes Harz enthalten.

7. Vliesstoff nach einem der Ansprüche 1 bis 6, wobei
ein durchschnittlicher Faserdurchmesser der kontinuierlichen Fasern 15 µm oder mehr und 30 µm oder weniger beträgt und ein durchschnittliches Flächengewicht des Vliesstoffs 10 g/m² oder mehr und 40 g/m² oder weniger beträgt.

8. Vliesstoff nach einem der Ansprüche 1 bis 7, wobei
die erste Richtung in einer Längsrichtung des absorbierenden Artikels angeordnet ist.

9. Vliesstoff nach einem der Ansprüche 1 bis 7, wobei
die erste Richtung in einer Breitenrichtung des absorbierenden Artikels angeordnet ist.

10. Vliesstoff nach einem der Ansprüche 1 bis 9, wobei
jeder der Vielzahl von Rippenteilen eine Vielzahl von vorstehenden Teilen (22) aufweist, die zu jedem der Vielzahl von benachbarten Rillenteilen vorstehen und nebeneinander mit Abständen entlang der ersten Richtung angeordnet sind.

11. Vliesstoff nach einem der Ansprüche 1 bis 10, wobei
die Faserausrichtung von jedem der Vielzahl von Rippenteilen entlang der ersten Richtung höher ist als die Faserausrichtung von jedem der Vielzahl von Rillenteilen entlang der ersten Richtung.

12. Vliesstoff nach einem der Ansprüche 1 bis 11, wobei
der Vliesstoff ein Spinnvliesstoff ist.

13. Verfahren zur Herstellung des Vliesstoffs nach einem der Ansprüche 1 bis 12, wobei das Verfahren Folgendes umfasst:
einen Schritt, während des Ansaugens der kontinuierlichen Fasern von einer unteren Seite eines Drahtgitters, das eine Transportrichtung, eine Querrichtung und eine Höhenrichtung aufweist, die rechtwinklig zueinander vorliegen, des Ablegens der kontinuierlichen Fasern auf dem Drahtgitter; und
einen Schritt des thermischen Verbindens der kontinuierlichen Fasern, die auf dem Drahtgitter abgelegt sind, wobei das Drahtgitter Folgendes einschließt:
einen Gitterhauptkörper (28) und
eine Vielzahl von Stegen (30), die sich entlang der Transportrichtung erstrecken, auf dem Gitterhauptkörper mit vorgegebenen Abständen in der Querrichtung angeordnet sind und in Richtung der Höhenrichtung vorstehen.

14. Verfahren zur Herstellung des Vliesstoffs nach Anspruch 13, wobei
eine Höhe des Stegs 1,0 mm oder mehr beträgt.

15. Verfahren zur Herstellung des Vliesstoffs nach Anspruch 13 oder 14, wobei
eine Gesamtlänge von Längen von jedem der Vielzahl von Stegen in der Querrichtung 50 % oder weniger einer Länge des Gitterhauptkörpers in der Querrichtung beträgt.

16. Verfahren zur Herstellung des Vliesstoffs nach einem der Ansprüche 13 bis 15, wobei
jeder der Vielzahl von Stegen eine Vielzahl von gewölbten Teilen aufweist, die in der Querrichtung gewölbt sind.

17. Verfahren zur Herstellung des Vliesstoffs nach einem der Ansprüche 13 bis 16, wobei
ein Außendurchmesser eines Drahts, der das Drahtgitter bildet, kleiner ist als eine Höhe des Stegs.

18. Verfahren zur Herstellung des Vliesstoffs nach einem der Ansprüche 13 bis 17, wobei
die kontinuierlichen Fasern auf dem Drahtgitter bei einer Fasergeschwindigkeit von 1000 m/min oder mehr und 4500 m/min oder weniger abgelegt werden.

## Revendications

1. Tissu non tissé pour un article absorbant, ayant une première direction (x), une deuxième direction (y) et une direction allant dans le sens de l'épaisseur (z) qui sont orthogonales les unes par rapport aux autres et étant formé à partir de fibres continues, le tissu non tissé comportant :
une pluralité de parties formant nervure (12) qui s'étendent le long de la première direction ; et
une pluralité de parties formant rainure (14) qui s'étendent le long de la première direction et ont une masse surfacique inférieure à celle de la pluralité de parties formant nervure, dans lequel
chacune de la pluralité de parties formant nervure et chacune de la pluralité de parties formant rainure sont situées de manière alternée dans la deuxième direction, et
chacune de la pluralité de parties formant nervure et chacune de la pluralité de parties formant rainure ont une pluralité de parties d'assemblage dans lesquelles les intersections des fibres continues sont assemblées thermiquement, **caractérisé en ce que**
chacune de la pluralité de parties formant nervure a une pluralité de parties évidées (16) qui sont évidées dans la direction allant dans le sens de l'épaisseur, et une base (18) formée à partir des fibres continues au niveau d'une partie inférieure de chacune de la pluralité de parties évidées.

2. Tissu non tissé selon la revendication 1, dans lequel
le tissu non tissé est pour une feuille perméable aux liquides de l'article absorbant.

3. Tissu non tissé selon la revendication 1, dans lequel
le tissu non tissé est pour une feuille extérieure de l'article absorbant.

4. Tissu non tissé selon la revendication 1, dans lequel,
chacune de la pluralité de parties formant nervure a une partie en forme de touffe (20) assemblée au niveau de la pluralité de parties d'assemblage dans un état dans lequel les fibres continues sont orientées le long de la première direction sur un côté d'au moins une partie de la pluralité de parties évidées dans la deuxième direction.

5. Tissu non tissé selon la revendication 1 ou la revendication 4, dans lequel
une épaisseur de chacune de la pluralité de parties formant rainure est inférieure à une épaisseur de la base.

6. Tissu non tissé selon l'une quelconque des revendications 1 à 5, dans lequel
les fibres continues sont des fibres conjuguées contenant une résine à base d'oléfine.

7. Tissu non tissé selon l'une quelconque des revendications 1 à 6, dans lequel
un diamètre moyen des fibres continues est de 15 µm ou plus et de 30 µm ou moins, et une masse surfacique moyenne du tissu non tissé est de 10 g/m² ou plus et de 40 g/m² ou moins.

8. Tissu non tissé selon l'une quelconque des revendications 1 à 7, dans lequel
la première direction est agencée dans une direction allant dans le sens de la longueur de l'article absorbant.

9. Tissu non tissé selon l'une quelconque des revendications 1 à 7, dans lequel
la première direction est agencée dans une direction allant dans le sens de la largeur de l'article absorbant.

10. Tissu non tissé selon l'une quelconque des revendications 1 à 9, dans lequel
chacune de la pluralité de parties formant nervure a une pluralité de parties saillantes (22) qui font saillie vers chacune de la pluralité de parties formant rainure adjacentes et sont agencées côte à côte avec des espaces le long de la première direction.

11. Tissu non tissé selon l'une quelconque des revendications 1 à 10, dans lequel
l'orientation des fibres de chacune de la pluralité de parties formant nervure le long de la première direction est supérieure à l'orientation des fibres de chacune de la pluralité de parties formant rainure le long de la première direction.

12. Tissu non tissé selon l'une quelconque des revendications 1 à 11, dans lequel
le tissu non tissé est un tissu non tissé par filage direct.

13. Procédé de fabrication du tissu non tissé selon l'une quelconque des revendications 1 à 12, le procédé comportant :
une étape consistant, tout en aspirant les fibres continues depuis un côté inférieur d'un treillis métallique ayant une direction allant dans le sens du transport, une direction transversale et une direction allant dans le sens de la hauteur qui sont orthogonales les unes par rapport aux autres, à déposer les fibres continues sur le treillis métallique ; et
une étape consistant à assembler thermiquement les fibres continues déposées sur le treillis métallique, dans lequel le treillis métallique comprend
un corps principal de treillis (28), et
une pluralité de nervures (30) qui s'étendent le long de la direction allant dans le sens du transport, qui sont agencées sur le corps principal de treillis avec des espaces prédéterminés dans la direction transversale, et qui font saillie vers la direction allant dans le sens de la hauteur.

14. Procédé de fabrication du tissu non tissé selon la revendication 13, dans lequel
la hauteur de la nervure est de 1,0 mm ou plus.

15. Procédé de fabrication du tissu non tissé selon la revendication 13 ou la revendication 14, dans lequel
une longueur totale des longueurs de chacune de la pluralité de nervures dans la direction transversale représente 50 % ou moins d'une longueur du corps principal de treillis dans la direction transversale.

16. Procédé de fabrication du tissu non tissé selon l'une quelconque des revendications 13 à 15, dans lequel
chacune de la pluralité de nervures a une pluralité de parties bombées qui se bombent dans la direction transversale.

17. Procédé de fabrication du tissu non tissé selon l'une quelconque des revendications 13 à 16, dans lequel
un diamètre extérieur d'un fil qui forme le treillis métallique est inférieur à une hauteur de la nervure.

18. Procédé de fabrication du tissu non tissé selon l'une quelconque des revendications 13 à 17, dans lequel
les fibres continues sont déposées sur le treillis métallique à une vitesse de fibre de 1000 m/min ou plus et de 4500 m/min ou moins.
